# EUROPÄISCHE PATENTSCHRIFT

(11) **EP 0 659 410 B1**
(45) Veröffentlichungstag und Bekanntmachung des Hinweises auf die Patenterteilung: **17.10.2001**
(21) Anmeldenummer: 95101189.9
(22) Anmeldetag: 04.07.1991
(51) Int. Cl.: A61K 31/44

(54) **Verwendung des Wirkstoffs Flupirtin zur Herstellung eines Arzneimittels zur Bekämpfung von Muskelverspannungen**
Use of the compound flupirtin for the preparation of a medicament against muscle bracing
Utilisation du composé flupirtin pour la préparation d'un medicament pour lutter contre la raideur musculaire

(30) Priorität: 14.07.1990 DE 4022442
(43) Veröffentlichungstag der Anmeldung: 28.06.1995
(62) Teilanmeldung aus: 91111124.3
(73) Patentinhaber: ASTA Medica Aktiengesellschaft, 01277 Dresden (DE)
(72) Erfinder: Lobisch, Michael, Dr., D-64372 Ober-Ramstadt (DE); Venhaus, Ralph, Dr., Hewitt, NY 07421 (US); Nickel, Bernd, Dr., D-64367 Mühltal (DE); Szelenyi, Istvan, Prof., D-90571 Schwaig (DE); Engel, Jürgen, Prof., D-63755 Alzenau (DE); Emig, Peter, Dr., D-61138 Niederdorffelden (DE)
(74) Vertreter: Decker, Karl-Ludwig

(56) Entgegenhaltungen:
- EP-A- 0 189 788
- DE-A- 3 604 575
- ARZNEIMITTEL-FORSCHUNG, Bd.35, Nr.1, 1985 Seiten 44 - 55 V.V. JAKOVLEV ET AL: 'Allgemeine pharmakologische Untersuchungen mit dem Analgetikum Flupirtin'
- ARZNEIMITTEL-FORSCHUNG, Bd.40(II), Nr.8, 1990 Seiten 909 - 911 B. NICKEL ET AL: 'Einfluss von Flupirtin, verschiedener Analgetika und Muskelrelaxantien auf den Skelettmuskeltonus wacher Ratten'
- FORTSCHR. MED., Bd.109, Nr.6, 28. Februar 1991 Seiten 158 - 160 R. WÖRZ: 'Flupirtin bei chronischen myofasziellen Schmerzzuständen'
- PHARMAZEUTISCHE ZEITUNG, Bd.136, Nr.30, 28. Februar 1991 Seite 28 J. BRÜGGMANN: 'Flupirtin bei Verspannungen der Muskulatur'

## Beschreibung

Der Arzneimittelwirkstoff Flupirtin (chemische Bezeichnung 2-Amino-3-carbethoxyamino-6-(4-fluorbenzylamino)-pyridin ist ein Analgetikum. Die Herstellung dieser Verbindung und deren physiologisch verwendbare Salze sind in der deutschen Patentschrift DE 17 95 858 sowie in DE 31 33 519 beschrieben.

Die aus der Literatur (Jakovlev et.al., Arzneimittelforschung/ Drug Research 35(1); 44-55,1985) bekannten Ergebnisse wurden an gesunden, nicht vorbehandelten Mäusen erhoben, die einen normalen Tonus der quergestreiften Muskulatur aufweisen. Die für Flupirtin gezeigten mutmaßlichen tonusveränderten Effekte an der Skelettmuskulatur wurden auch für andere Substanzen wie Phenacetin und sogar für Codeinphosphat gezeigt(Tabelle 2). Das Herabfallen der Mäuse in dem durchgeführten Schräggittertest ist hier jedoch ein Zeichen einer allgemeinen Sedierung bzw. motorischen Koordinationsstörung, die bei im Nebenwirkungsbereich liegenden hohen Dosen auch bei anderen Substanzen beobachtet wurden. Die mit diesem Versuch erzielten Ergebnisse sind keineswegs mit einer skelettmuskelrelaxierenden Wirkung von Flupirtin gleichzusetzen, auch wenn dies die Kapitelüberschrift der Publikation von Jakovlev fälschlich suggeriert. Weder Phenacetin noch Codein besitzen derartig aufgezeigte Eigenschaften, Codein führt im Gegenteil bekanntlich zu einem Anstieg des Muskeltonus.

Der Erfindung liegt nun die Aufgabe zugrunde, ein Arzneimittel mit skelettmuskelentspannender Wirkung zur Verfügung zu stellen, welches für die Behandlung von Krankheitsbildern, Krankheitssymptomen und Erkrankungen geeignet ist, die auf Muskelverspannungen beruhen oder eine Folge von solchen Muskelverspannungen sind.

Es wurde nun gefunden, daß Flupirtin eine starke skelettmuskelentspannende Wirkungen besitzt, wenn eine krankhafte Muskelrigidität vorliegt.

Flupiritin ist deshalb für die Behandlung von Krankheitsbildern, Krankheitssymptomen und Erkrankungen geeignet, die auf Muskelverspannungen beruhen oder eine Folge von solchen Muskelverspannungen sind.

### Versuchsmethode

Als Versuchstiere wurden weibliche SIV 50-Ratten, 160 g bis 200 g, eingesetzt. Die Tiere wurden bei Raumtemperaturen von 20°C bis 24°C, einer relativen Luftfeuchtigkeit von 60 % bis 70 % und Hell-Dunkel-Zeiten von 12 zu 12 Stunden gehalten. Futter (Atromin®, Altrogge) und Wasser standen adlibitum zur Verfügung. Flupirtin wurde als Maleat eingesetzt.

Die muskelentspannende Wirkung wurde durch kontinuierliche Messung des Widerstandes des Flexor- und Extensor-Muskels am Hinterbein der Ratten gemessen. Dazu wurde der Fuß des rechten Hinterbeins der Ratte durch einen elektronisch angesteuerten Stellmotor in einem zeitlichen Abstand von 30 Sekunden schnell nach oben und danach nach unten bewegt. Die Reflexreaktion von Flexor und Extensor der Ratten, hervorgerufen durch die zwanghafte Bewegung des Rattenfußes, wurde auf einen Kraftaufnehmer übertragen und rechnergestützt (IBM/PC) ausgewertet.

Parallel zur on-line-Auswertung wurden die Daten auf einem hierfür üblichen Schreiber mitgeschrieben und gleichzeitig auf einem Magnetband abgespeichert. Vor Versuchsbeginn wurde die Versuchseinheit durch Auflegen eines geeichten 50g-Gewichtes auf den Kraftaufnehmer kalibriert. Danach wurden die Tiere in die Versuchsanordung gesetzt und hatten 30 Minuten Zeit, sich zu adaptieren. Nach Ermittlung des Ausgangswiderstands von Flexor und Extensor der Ratten (Kontrollwerte) wurde Flupirtin-Maleat intraperitoneal appliziert und der Einfluß der Verbindungen auf Flexor und Extensor über 90 Minuten, wobei eine Mitteilung über jeweils 10 Minuten erfolgte, kontinuierlich registriert, abgespeichert und on-line ausgewertet.

Die Unterschiede zwischen den Mittelwerten der Versuchstiergruppen wurden mit dem t-Test nach Student auf Signifikanz geprüft.

Nach intraperitonealer Gabe von Flupirtin im analgetisch wirksamen Dosenbereich wurde ein muskelentspannender Effekt festgestellt, wobei in dem untersuchten Dosenbereich bei den mit Flupirtin behandelten Tieren keine zentralen Nebenwirkungen wie Ataxie beziehungsweise Verminderung der Spontanmotilität beobachtet wurden.

Beispielsweise wird bei obengenannter Versuchsmethode bei einer Dosis von 1 mg/Körpergewicht kg Ratte die medikamentös induzierte Rigidität der Skelettmuskulatur in der wachen Ratte um 50 % reduziert.

Die niedrigste, bereits wirksame Dosis in dem oben angegebenen Tierversuch ist beispielsweise
1 mg/kg oral
0,1 mg/kg intraperitoneal

Als allgemeiner Dosisbereich für die Wirkung (Tierversuch wie oben) kommt beispielsweise in Frage:
1 - 100 mg/kg oral, insbesondere 10 bis 40 mg/kg
0,1 - 30 mg/kg intraperitoneal, insbesondere 1 bis 20 mg/kg

Zu anderen Wirkstoffen gleicher Wirkungsrichtung zeigt das Flupirtin insbesondere folgende Unterschiede: Flupirtin ruft keine Abhängigkeit hervor und besitzt eine starke analgetische Wirkung.

Die Wirkung von Flupirtin und verschiedenen Vergleichssubstanzen auf die reserpininduzierte Rigidität geht aus der folgenden Tabelle hervor.

| Rigiditätsabnahme (-) bzw. -zunahme (+) | | | |
|---|---|---|---|
| Substanz | Dosis mg/kg i.p* | Flexor %Wirkung | Extensor %Wirkung |
| Flupirtin | 2.5 | -23 | -17 |
| | 5.0 | -53 | -48 |
| | 10.0 | -81 | -87 |
| | | | |
| (-)-Deprenyl | 1 | -18 | -14 |
| | | | |
| Morphin | 2.5 | + 8 | + 4 |
| | 5.0 | +31 | +49 |
| | 10.0 | +74 | +80 |
| | | | |
| Codein | 25 | +11 | + 9 |
| | 50 | +21 | +19 |
| | 100 | +48 | +52 |
| | | | |
| Phenacetin | 50 | - 5 | + 4 |
| | 100 | + 8 | + 6 |
| | 200 | -10 | - 8 |
| | 400 | +11 | + 9 |
| | | | |
| Ibuprofen | 20 | - 6 | + 2 |
| | 40 | + 8 | - 9 |
| | 80 | + 3 | - 6 |
| | | | |
| Indomethacin | 2,5 | + 8 | - 2 |
| | 5.0 | + 6 | + 9 |
| | 10.0 | - 5 | - 8 |
| | | | |
| Amitriptylin | 5.0 | - 9 | - 6 |
| | 10.0 | + 8 | + 2 |
| | 20.0 | -11 | + 1 |

| | | | |
|---|---|---|---|
| *i.p.=intraperitoneal | | | |

| Rigiditätsabnahme (+) bzw. -abnahme (-) | | | |
|---|---|---|---|
| Substanz | Dosis mg/kg p.o. * | Flexor %Wirkung | Extensor %Wirkung |
| Flupirtin | 5.0 | -13 | -16 |
| | 10.0 | -43 | -35 |
| | 20.0 | -69 | -71 |
| | 40.0 | -88 | -91 |
| | | | |
| Morphin | 10.0 | + 6 | + 9 |
| | 20.0 | +27 | +31 |
| | 40.0 | +54 | +69 |
| | | | |
| Codein | 50 | + 8 | + 4 |
| | 100 | +25 | +21 |
| | 200 | +53 | +59 |
| | | | |
| Phenacetin | 200 | - 8 | + 1 |
| | 400 | + 4 | - 6 |
| | 800 | + 7 | - 5 |
| | 1000 | -12 | + 6 |
| | | | |
| Ibuprofen | 40 | -12 | -10 |
| | 80 | + 9 | + 5 |
| | 160 | - 7 | - 9 |
| | | | |
| Indomethacin | 5.0 | + 4 | + 1 |
| | 10.0 | - 1 | - 5 |
| | 20.0 | + 8 | -11 |
| | | | |
| Amitriptylin | 10.0 | + 2 | - 3 |
| | 20.0 | +12 | + 9 |
| | 40.0 | + 6 | - 11 |

| | | | |
|---|---|---|---|
| *per oral | | | |

Diese Rigiditätsabnahme wird durch die bereits beschriebene kontinuierliche Messung des Widerstandes des Flexor- und Extensor-Muskels am Hinterbein der Ratte bestimmt. Der einzige Unterschied bei der Bestimmung der Rigiditätsabnahme besteht darin, daß den Tieren 16 Stunden vor Versuchsbeginn Reserpin in einer Dosis von 2,5 mg/kg Ratte intraperitoneal appliziert wird. Das Reserpin erzeugt die Rigidität. Die Abnahme beziehungsweise Aufhebung dieser Rigidität durch Flupirtin wird dann wie angegeben bestimmt.

Indikationen, für die das Flupirtin der erfindungsgemäßen neuen Wirkung in Betracht kommen kann: Alle Erkrankungen, die mit Muskelverspannung (Rigidität) und deren Folgeerscheinungen verbunden sind wie zum Beispiel Neuralgien, Arthritis, Arthrose, chronischer oder episodischer Spannungskopfschmerz, postoperative Behinderungen, generalisierte Tendomyopathien, Insertionstendopathien, Parkinson-Erkrankungen einhergehenden Rigidität.
Kontraindikationen: Myasthenia gravis.

Die pharmazeutischen Zubereitungen enthalten im allgemeinen zwischen 50 mg bis 500 mg, vorzugsweise 100 mg bis 200 mg Flupirtin-Base.

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen.

Die flüssige Anwendungsformen kommen zum Beispiel in Frage: Ölige oder alkoholische beziehungsweise wässrige Lösungen sowie Suspensionen und Emulsionen. Bevorzugte Anwendungsformen sind Kapseln oder Tabletten, die zwischen 100 mg und 200 mg oder Lösungen, die zwischen 0,1 bis 10 Gewichts% Flupirtin enthalten.

Die Einzeldosis an Flupirtin-Base kann beispielsweise liegen
a) bei oralen Arzneiformen zwischen 70 mg und 300 mg, vorzugsweise 100 mg bis 200 mg,
b) bei parenteralen Arzneiformen (zum Beispiels intravenös, intramuskulär) zwischen 8 mg und 80 mg, vorzugsweise 10 mg und 100 mg.

Die Dosen sind jeweils bezogen auf die freie Flupirtinbase.

Beispielsweise können 3 mal täglich 1 bis 2 Kapseln oder Tabletten mit einem Gehalt von 50 mg bis 200 mg wirksamer Substanz empfohlen werden.

Die akute Toxizität des Flupirtins an der Maus (ausgedrückt durch die LD 50 mg/kg; Methode nach Miller und Tinter: Proc. Soc. Exper. Biol. a. Med. 57 (1944) 261) liegt beispielsweise bei oraler Applikation zwischen 600 mg/kg und 800 mg/kg (beziehungsweise oberhalb 500 mg/kg).

Arzneimittel zur Behandlung von Erkrankungen und Krankheitssymptomen, die auf Muskelverspannungen beruhen oder eine Folgeerscheinung von Muskelverspannungen sind, werden hergestellt, in dem Flupirtin oder dessen therapeutisch verwertbare Salze mit üblichen pharmazeutischen Träger-, Hilfs- und/oder Verdünnungsmitteln zu einem Arzneimittel formuliert wird.

Flupirtin oder ein pharmazeutisch verwendbares Salz des Flupirtins wird zusammen mit üblichen Träger- und/oder Verdünnungs-beziehungsweise Hilfsstoffen bei Temperaturen zwischen 0 und 120°C, vorzugsweise 20 und 80°C vermischt beziehungsweise homogenisiet und gegebenenfalls die so erhaltene Mischung zur Herstellung von Zubereitungen, die in der Dosierungseinheit 50 bis 500 mg Flupirtin oder ein pharmazeutisch verwendbares Salz des Flupirtins enthalten, in Hohlzellen entsprechender Größe abgefüllt oder granuliert und dann gegebenenfalls unter Zusatz von weiteren üblichen Hilfsstoffen zu Tabletten verpreßt oder in Kapseln abgefüllt.

Flupirtin oder ein pharmazeutisch verwendbares Salz des Flupirtins kann mit einem oder mehreren der folgenden Stoffe: Stärke, Cyclodextrin, Harnstoff, Cellulose, Lactose, Formalin-Casein, modifizierte Stärke, Magnesiumstearat, Calciumhydrogenphosphat, Kieselsäure, Talkum, Phenoxyethanol vermischt, die erhaltene Mischung gegebenenfalls mit einer wässrigen Lösung, die als Bestandteil mindestens Gelatine, Stärke, Polyvinylpyrrolidon, Vinylpyrrolidon-Vinylacetat-Copolymerisat und/oder Polyoxyethylen-sorbitanmonooleat enthält, granuliert, das Granulat gegebenenfalls mit einem oder mehreren der oben genannten Hilfsstoffe homogenisiert, und diese Mischung zu Tabletten verpreßt oder in Kapseln abgefüllt werden, wobei die Tabletten oder Kapseln in der Dosierungseinheit jeweils 50 bis 500 mg Wirkstoff Flupirtin oder ein Salz hiervon enthalten.

Desweiteren können ein oder mehrere der folgenden Hilfsstoffe: Sojalecitin, Oxynex, Phenoxyethanol bei Temperaturen zwischen 31 und 65°C in geschmolzenem Hartfett oder anderen Fettsäureglyceride enthaltenen Mischungen suspendiert und homogenisiert und anschließend die Mischung in Hohlzellen ausgegossen oder in Kapseln abgefüllt werden, wobei die Dosierungseinheit 50 bis 500 mg Wirkstoff Flupirtin oder ein Salz hiervon enthält.

Flupirtin oder ein pharmazeutisch verwendbares Salz des Flupirtins kann bei einer Temperatur zwischen 20 bis 120°C, gegebenenfalls in Gegenwart eines oder mehrerer Emulatoren und/oder Komplexbildnern mit mindestens einem der folgenden Stoffe homogenisiert und/oder emulgiert werden: Wasser, Glycerin, Paraffin, Vaseline, alipatischer Alkohol mit 12 bis 25 C-Atomen, alipatische Monocarbonsäure mit 15 bis 20 C-Atomen, Sormitanmonopalmitat, Polyoxyethylenpolyolfettsäureester, ein- oder mehrwertiger niedrigmolekularer alipatischer Alkohol, Fettsäureglycerid, Wachs, Silikon, Polyethylenglykol, Siliziumdioxid.

Flupirtin oder ein pharmazeutisch verwendbares Salz des Flupirtins wird gegebenenfalls in Anwesenheit eines Komplexbildners und/oder eines Emulgators, bei Temperaturen zwischen 30 und 100°C in Wasser, physiologisch unbedenklichen Alkoholen, Polyglykolen, Polyglykolderivaten, Dimethylsulfoxid, Triglyceriden, Partialestern des Glycerins, Paraffinen oder Ölen oder Mischungen hiervon auflöst, und gegebenenfalls die erhaltene Lösung mit soviel der genannten Lösungsmittel aufgefüllt, daß die Endlösung, Endsuspension oder Endmulsion 0,1 - 10 Gewichtsprozent an Wirkstoff Flupirtin enthält.

Als weitere Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angeben sind:
Ullmanns Encyklopädie der technischen Chemie, Band 4 (1953), Seite 1 bis 39;
Journal of Pharmaceutical Sciences, Band 52 (1963), Seite 918 und ff.;
H.v.Czetsch-Lindenwald; Hilfsstoffe für Pharmazie und angrenzende Gebiete; Pharm. Ind., Heft 2 (1961), Seite 72 und ff.;
Dr. H.P. Fiedler, Lexikon der Hilfsstoffe für Pharmazie, Kosmetik und angrenzende Gebiete Cantor KG. Aulendorf in Württemberg 1981.

Beispiele hierfür sind Gelatine, natürliche Zucker wie Rohrzucker oder Milchzucker, Lecithin, Pektin, Stärke (zum Beispiel Maisstärke), Cyclodextrine und Cyclodextrinderivate, Polyvinylpyrrolidon, Polyvinylacetat, Gelatine, Gummi arabicum, Alginsäure, Tylose, Talkum Lycopodium, Kieselsäure (zum Beispiel kolloidale), Cellulose, Cellulosederivate (zum Beispiel Celluloseether, bei denen die Cellulose-Hydroxygruppen teilweise mit niederen gesättigten aliphatischen Alkoholen und/oder niederen gesättigten aliphatischen Oxyalkoholen verethert sind, zum Beispiel Methyloxypropylcellulose, Methylcellulose, Hydroxypropylmethylcellulose, Hydroxypropylmethylcellulosephthalat); Fettsäuren sowie Magnesium-, Calcium- oder Aluminiumsalze von Fettsäuren mit 12 bis 22 C-Atomen,

insbesondere der gesättigten (zum Beispiel Stearate), Emulgatoren, Öle und Fette, insbesondere pflanzliche (zum Beispiel Erdnußöl, Rizinusöl, Olivenöl, Sesamöl, Baumwollsaatöl, Maisöl, Weizenkeimöl, Sonnenblumensamenöl, Kabeljau-Leberöl, jeweils auch hydriert; Mono-, Di- und Triglyceride von gesättigten Fettsäuren C₁₂H₂₄O₂ bis C₁₈H₃₆O₂ und deren Gemische, pharmazeutisch verträgliche ein- oder mehrwertige Alkohole und Polyglykole wie Polyethylenglykole sowie Derivate hiervon, Ester von aliphatischen gesättigten oder ungesättigten Fettsäuren (2 bis 22 C-Atome, insbesondere 10 - 18 C-Atome) mit einwertigen aliphatischen Alkoholen (1 bis 20 C-Atome) oder mehrwertigen Alkoholen wie Glykolen, Glycerin, Diethylenglykol, Pentaerythrit, Sorbit, Mannit und so weiter, die gegebenenfalls auch verethert sein können, Ester der Zitronensäure mit primären Alkoholen, Essigsäure, Benzylbenzoat, Dioxolane, Glyzerinformale, Tetrahydrofurfurylalkohol, Polyglykolether mit C₁-C₁₂-Alkoholen, Dimethylacetamid, Lactamide, Lactate, Ethylcarbonate, Silicone (insbesondere mittelviskose Polydimethylsiloxane), Calciumcarbonat, Natriumcarbonat, Calciumphosphat, Natriumphosphat, Magnesiumcarbonat und ähnliche.

Als weitere Hilfsstoffe kommen auch Stoffe in Frage, die den Zerfall bewirken (sogenannte Sprengmittel) wie: quervernetztes Polyvinylpyrrolidon, Natriumcarboxymethylstärke, Natriumcarboxymethylcellulose oder mikrokristalline Cellulose. Ebenfalls können bekannte Hüllstoffe verwendet werden. Als solche kommen beispielsweise in Frage: Polymerisate sowie Copolymerisate der Acrylsäure und/oder Methacrylsäure und/oder deren Ester; Copolymerisate aus Acryl- und Methacrylsäureestern mit einem geringen Gehalt an Ammoniumgruppen (zum Beispiel Eudragit® RS), Copolymerisate aus Acryl- und Methacrylsäureestern und Trimethylammoniummethacrylat (zum Beispiel Eudragit®RL); Polyvinylacetat; Fette, Öle, Wachse, Fettalkohole; Hydroxypropylmethylcellulosephthalat oder -acetatsuccinat; Cellulose-, Stärke- sowie Polyvinylacetatphthalat; Carboxymethylcellulose; Methylcellulose-phthalat, -succinat, -phthalatsuccinat sowie -phthalatsäurehalbester; Zein; Ethylcellulose sowie -succinat; Schellack, Gluten; Ethylcarboxyethylcellulose; Ethacrylat-Maleinsäureanhydrid-Copolymer; Maleinsäureanhydrid-Vinylmethylether-Copolymer; Styrol-Maleinsäure-Copolymerisate; 2-Ethyl-hexylacrylatmaleinsäureanhydrid; Crotonsäure-Vinylacetat-Copolymer; Glutaminsäure/Glutaminsäureester-Copolymer; Acarboxymethylethyl-celluloseglycerinmonooctanoat; Celluloseacetatsuccinat; Polyarginin.

Als Plastifizierungsmittel für Hüllstoffe können in Frage kommen:

Citronen- und Weinsäureester (Acetyltriethyl-, Acetyltributyl-, Tributyl-, Triethyl-citrat); Glycerin und Glycerinester (Glycerindiacetat, -triacetat, acetylierte Monoglyceride, Rizinusöl); Phthalsäureester (Dibutyl-, Diamyl-, Diethyl-, Dimethyl-, Dipropyl-phthalat), D-(2-Methoxy- oder ethoxyethyl)-phthalat, Ethylphthalyl-, Butylphthalylethyl- und Butylglycolat; Alkohole (Propylenglycol, Polyethylenglycol verschiedener Kettenlängen), Adipate (Di-ethyl-adipat, Di(2-Methoxy- oder ethoxyethyladipat); Benzophenon; Diethyl- und Dibutylsebacat, -succinat, -tartrat; Diethylenglycoldipropionat; Ethylenglykoldiacetat, -dibutyrat, -dipropionat; Tributylphosphat, Tributyrin; Polyethylenglykolsorbitanmonooleat (Polysorbate wie Polysorbat 80); Sorbitanmooleat.

Zur Herstellung von Lösungen oder Suspensionen kommen beispielsweise Wasser oder physiologisch verträgliche organische Lösungsmittel in Frage, wie zum Beispiel Ethanol, Propanol, Isopropanol, 1,2-Propylenglykol, Polyglykole und deren Derivate, Dimethylsulfoxid, Fettalkohole, Triglyceride, Partialester des Glycerins, Paraffine und ähnliche.
Für injizierbare Lösungen oder Suspensionen kommen zum Beispiel nicht-toxische parenteral verträgliche Verdünnungsmittel oder Lösungsmittel in Frage, wie zum Beispiel: Wasser, 1,3-Butandiol, Ethanol, 1,2-Propylenglykol, Polyglykole in Mischung mit Wasser, Ringer's Lösung, isotonische Kochsalzlösung oder auch gehärtete Öle einschließlich synthetischer Mono- oder Diglyceride oder Fettsäuren wie Oleinsäure.

Bei der Herstellung der Zubereitungen können bekannte und übliche Lösungsvermittler, beziehungsweise Emulgatoren, verwendet werden. Als Lösungsvermittler und Emulgatoren kommen beispielsweise in Frage:
Polyvinylpyrrolidon, Sorbitanfettsäureester wie Sorbitantrioleat, Phosphatide, wie Lecithin, Acacia, Traganth, polyoxyethyliertes Sorbitanmonooleat und andere ethoxylierte Fettsäureester des Sorbitan, polyoxyethylierte Fett, polyoxyethylierte Oleotriglyceride, linolisierte Oleotriglyceride, Polyethylenoxyd-Kondensationsprodukte von Fettalkoholen, Alkylphenolen oder Fettsäuren oder auch 1-Methyl-3-(2-hydroxyethyl)-imidazolidon-(2). Polyoxyethyliert bedeutet hierbei, daß die betreffenden Stoffe Polyoxyethylenketten enthalten, deren Polymerisationsgrad im allgemeinen zwischen 2 bis 40 und insbesondere zwischen 10 bis 20 liegt.
Solche polyoxyethylierten Stoffe können beispielsweise durch Umsetzung von hydroxylgruppenhaltigen Verbindungen (beispielsweise Mono- oder Diglyceride oder ungesättigte Verbindungen wie zum Beispiel solche, die Ölsäurereste enthalten) mit Ethylenoxyd erhalten werden (zum Beispiel 40 Mol Ethylenoxy pro Mol Glycerid).
Beispiele für Oleotriglyceride sind Olivenöl, Erdnußöl, Rizinusöl, Sesamöl, Baumwollsaatöl, Maisöl.
Siehe auch Dr. H.P. Fiedler "Lexikon der Hilfstoffe für Pharmazie, Kosmetik und angrenzende Gebiete" S. 191-195.

Darüber hinaus ist der Zusatz von Konservierungsmitteln, Stabilisatoren, Puffersubstanzen, zum Beispiel Calciumhydrogenphosphat, kolloidales Aluminiumhydroxyd, Geschmackskorrigentien, Süßmitteln, Farbstoffen, Antioxydantien und Komplexbildnern (zum Beispiel Ethylendiaminotetraessigsäure) und dergleichen möglich. Gegebenenfalls ist zur Stabilisierung des Wirkstoffmoleküls mit physiologisch verträglichen Säuren oder Puffern auf einen pH-Bereich von ca. 3 bis 7 einzustellen. Im allgemeinen wird ein möglichst neutraler bis schwach saurer (bis pH 5) pH-Wert bevorzugt.

Zur Herstellung von dermal anzuwendenden Zubereitungen kommen in Frage die vorgenannten Substanzen und streichfähige oder flüssige Kohlenwasserstoffe wie Vaselin oder Paraffin oder Gele aus Paraffinkohlenwasserstoffen und Polyethylen, Fette und Öle pflanzlichen oder tierischen Ursprungs, die zum Teil auch hydriert sein können oder synthetische Fette wie Glyceride der Fettsäuren C₈-C₁₈, sodann Bienenwachs, Cetylpalmitat, Wollwachs, Wollwachsalkohole; Fettalkohole wie Cetylalkohol, Stearylalkohol, Polyethylenglykole vom Molekulargewicht 200 bis 20 000; flüssige Wachse wie Isopropylmyristat, Isopropylstearat, Äthyloleat; Emulgatoren wie Natrium-, Kalium-, Ammoniumsalze der Stearinsäure oder Palmitinsäure sowie Triethanolaminstearat, Alkalisalze der Ölsäure, Ricinolsäure, Salze von sulfurierten Fettalkoholen wie Natriumlaurylsulfat, Natriumcetylsulfat, Natriumstearylsulfat, Salze der Gallensäure, Sterole wie Cholesterol, Partialfettsäureester mehrwertiger Alkohole wie Ethylenglykolmonostearat, Partialfettsäureester des Sorbitans, Partialfettsäureester des Polyoxyethylensorbitans, Sorbitolether des Polyoxyethylens, Fettsäureester des Polyoxyethylens, Fettalkoholether des Polyoxyethylens, Fettsäureester der Saccharose, Fettsäureester des Polyglycerols, Lecithin.

Als Antioxydantien kommen beispielsweise Natriummetabisulfit, Ascorbinsäure, Gallussäure, Gallussäurealkylester, Butylhydroxyanisol, Nordihydroguajaretsäure, Tocopherole sowie Tocopherole + Synergisten (Stoffe, die Schermetalle durch Komplexbildung binden, beispielsweise Lecithin, Ascorbinsäure, Phosphorsäure) zur Anwendung. Der Zusatz der Synergisten steigert die antioxygene Wirkung der Tocopherole erheblich. Als Konservierungsmittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid und Formalinderivate in Betracht.

Die pharmazeutische und galenische Handhabung der erfindungsgemäßen Verbindungen erfolgt nach den üblichen Standardmethoden. Beispielsweise wird Flupiritin und die Hilfs- beziehungsweise Trägerstoffe durch Rühren oder Homogenisieren (zum Beispiel mittels üblicher Mischgeräte) gut vermischt, wobei im allgemeinen bei Temperaturen zwischen 20 und 80°C, vorzugsweise 20 bis 50°C, insbesondere bei Raumtemperatur gearbeitet wird.

Im übrigen wird auf das folgende Standardwerk verwiesen: Sucker, Fuchs, Speiser, Pharmazeutische Technologie, Thieme-Verlag Stuttgart, 1978.

Die Applikation kann auf die Haut oder Schleimhaut oder in das Körperinnere erfolgen, beispielsweise oral, enteral, pulmonal, rectal, nasal, vaginal, lingual, intravenös, intraarteriell, intrakardial, intramuskulär, intraperitoneal, intracutan, subcutan.

Bei den parenteralen Zubereitungsformen handelt es sich insbesondere um sterile beziehungsweise sterilisierte Erzeugnisse.

### Beispiele:

### Tabletten:

10 kg 2-Amino-3-carbethoxyamino-6-(4-fluor-benzylamino)-pyridin-mealeat werden mit 2,5 kg Calciumhydrogenphosphat und 2,5 kg Maisstärke gemischt und die Mischung mit einer Lösung von 1 kg Polyvinylpyrrolidon in 4 kg demineralisiertem Wasser in bekannter Weise granuliert. Nach Zumischen von 1,3 kg Maisstärke, 2 kg mikrokristalliner Cellulose, 0,6 kg Magnesiumstearat und 0,1 kg hochdispersem Siliciumdioxid werden Tabletten mit einem Gewicht von 200 mg, einem Durchmesser von 9 mm und einem Wölbungsradius von 10 mm mit Bruchkerbe gepresst. Die Bruchfestigkeit der Tabletten beträgt 80 N bis 100 N (Schleuniger-Bruchfestigtester). Die Zerfallzeit nach DAB 8 beträgt 5 Minuten.
Jede Tablette enthält 100 mg Wirkstoff.

### Kapseln:

Analog der zuvor beschriebenen Herstellungsweise für Tabletten wird eine Kapselfüllung hergestellt, die in Hartgelatine-Kapseln der passenden Größe abgefüllt wird. Füllmenge pro Kapsel: 200 mg.
Eine Kapsel enthält 100 mg Wirkstoff.

### Ölige Suspension mit 15 % Flupirtinmaleat

In 400 g mittelkettiger Triglyceriden (Handelsname: Miglyol 812/Hüls Troisdorf) werden 32,5 g hochdisperses, amorphes, hydrophobes Siliciumdioxid (Handelsnahme: Aerosil R 972/Degussa), 0,5 g mikronisiertes Saccharin-Natrium,1,5 g fein gemahlenes Natriumcyclamat, 0,1 g Eisenoxid, rot, 0,5 g Erdbeeraroma, 75 g Flupritinmaleat und 0,375 g Oxynex LM (E. Merck/Darmstadt) homogen suspendiert. Die Suspension wird mit mittelkettiger Triglyderiden auf 500 g aufgefüllt und gemischt.

Ein Gramm der Suspension enthält 150 mg Flupirtin.

### Injektionslösung

### Der Herstellungsgang gilt für einen Ansatz zu 20 Liter (= 6500 Ampullen):

### Herstellungsgang:

1. 10,0 1 Wasser werden auf 70°C erwärmt und nach Zugabe von 1562,0 g Gluconsäure-delta-Lacton wird die Lösung eine Stunde bei 70°C belassen. Die Lösung wird dabei mit Stickstoff begast.
2. In Lösung 1 werden 8000,0 g Polyethylenglykol Molekulargewicht 380 bis 420 eingewogen und die Lösung unter Stickstoffbegasung auf 70°C erwärmt.
3. 30,0 g Natriumdisulfit werden in 500,0 ml mit Stickstoff begastem Wasser gelöst.
4. Lösung 3 wird zu Lösung 2 gegeben.
5. 666,6 g Flupirtin-Base werden durch ein Sieb mit der Maschenweite 0,3 mm gesiebt und in Lösung 4 unter intensiver Stickstoffbegasung gelöst.
6. Lösung 5 wird abgekühlt und ad 20 Liter mit Stickstoff begastem Wasser aufgefüllt.
7. Lösung 6 wird durch ein Membranfilter der Porenweite 0,2 µm mit Glasfaservorfilter steril filtriert.
8. Inprozesskontrolle: Messung des Sauerstoffgehaltes von Lösung 7 mittels Sauerstoffelektrode. Messung des pH-Wertes von Lösung 7.
9. Lösung 7 wird unter aseptischen Bedingungen sowie unter Stickstoffbegasung in farblose Ampullen, Inhalt 3 ml, abgefüllt.

Eine Ampulle enthält 164,5 mg Flupirtin-Gluconat in 3 ml Lösung.

## Patentansprüche

1. Verwendung von Flupirtin oder dessen therapeutisch verwertbaren Salzen für die Herstellung eines Arzneimittels zur Behandlung von Erkrankungen und Krankheitssymptomen, die auf Muskelverspannungen beruhen oder eine Folge von Muskelverspannungen sind.

2. Verwendung von Flupirtin oder dessen therapeutisch verwertbaren Salzen nach Anspruch 1 bei der Behandlung von chronischem oder episodischem Spannungskopfschmerz.

3. Verwendung von Flupirtin oder dessen therapeutisch verwertbaren Salzen nach Anspruch 1 bei der Behandlung von Neuralgien.

4. Verwendung von Flupirtin oder dessen therapeutisch verwertbaren Salzen nach Anspruch 1 bei der Behandlung von Arthritis sowie Arthrose.

5. Verwendung von Flupirtin oder dessen therapeutisch verwertbaren Salzen nach Anspruch 1 bei der Behandlung von postoperativen Behinderungen.

## Claims

1. Use of flupirtine or its therapeutically utilizable salts for the production of a medicament for treating disorders and symptoms which are based on muscle tenseness or are a result of muscle tenseness.

2. Use of flupirtine or its therapeutically utilizable salts according to Claim 1 in the treatment of chronic or episodic tension headache.

3. Use of flupirtine or its therapeutically utilizable salts according to Claim 1 in the treatment of neuralgia.

4. Use of flupirtine or its therapeutically utilizable salts according to Claim 1 in the treatment of arthritis and arthrosis.

5. Use of flupirtine or its therapeutically utilizable salts according to Claim 1 in the treatment of post-operative disabilities.

## Revendications

1. Utilisation de flupirtine ou de ses sels thérapeutiquement utilisables pour la production d'un médicament pour le traitement de maladies et de symptômes de maladie qui proviennent de raideurs musculaires ou qui sont une conséquence de raideurs musculaires.

2. Utilisation de flupirtine ou de ses sels thérapeutiquement utilisables selon la revendication 1,
pour le traitement de maux de tête liés à la tension chroniques ou épisodiques.

3. Utilisation de flupirtine ou de ses sels thérapeutiquement utilisables selon la revendication 1,
pour le traitement de neuralgies.

4. Utilisation de flupirtine ou de ses sels thérapeutiquement utilisables selon la revendication 1,
pour le traitement de l'arthrite ainsi que de l'arthrose.

5. Utilisation de flupirtine ou de ses sels thérapeutiquement utilisables selon la revendication 1,
pour le traitement d'empêchements postopératoires.
